(19) **Europäisches Patentamt**

**European Patent Office**

**Office européen des brevets**

(11) **EP 1 074 251 A1**

(12) **EUROPEAN PATENT APPLICATION**
published in accordance with Art. 158(3) EPC

(43) Date of publication:
07.02.2001 Bulletin 2001/06

(51) Int. Cl.⁷: **A61K 9/70**, A61K 45/08,
A61K 31/135, A61K 31/14,
A61K 31/165, A61K 31/215,
A61K 31/415, A61K 31/445,
A61K 31/55

(21) Application number: 99913584.1

(22) Date of filing: 08.04.1999

(86) International application number:
PCT/JP99/01868

(87) International publication number:
WO 99/53906 (28.10.1999 Gazette 1999/43)

(84) Designated Contracting States:
**AT BE CH DE DK ES FI FR GB GR IE IT LI LU MC NL PT SE**

(30) Priority: 17.04.1998 JP 12275898

(71) Applicant:
**HISAMITSU PHARMACEUTICAL CO. INC.**
**Tosu-shi Saga 841-0017 (JP)**

(72) Inventors:
• **KURITA, Hisakazu**
**Hisamitsu Pharmaceu. Co., Inc.**
**Tsukuba-shi Ibaraki 305-0856 (JP)**

• **TATEISHI, Tetsuro**
**Hisamitsu Pharmaceu. Co., Inc.**
**Tsukuba-shi Ibaraki 305-0856 (JP)**
• **CHONO, Hideharu**
**Hisamitsu Pharmaceutical Co., Inc.**
**Tsukuba-shi Ibaraki 305-0856 (JP)**
• **HIGO, Naruhito**
**Hisamitsu Pharmaceutical Co., Inc.**
**Tsukuba-shi Ibaraki 305-0856 (JP)**

(74) Representative:
**Forstmeyer, Dietmar, Dr. rer. nat., Dipl.-Chem. et al**
**Boeters & Bauer,**
**Bereiteranger 15**
**81541 München (DE)**

(54) **ADHESIVE PREPARATIONS**

(57) Adhesive preparations with improved percutaneous absorption of physiologically active substances, in particular, matrix adhesive preparations containing a base drug salt and an organic acid salt having an mean diameter of from 0.1 to 100 μm are provided.

## Description

### TECHNICAL FIELD

The invention relates to a percutaneous absorption preparation which contains a salt of a base drug and is excellent in skin permeability of the drug.

### BACKGROUND ART

As administration methods for drugs, various methods such as oral administration, rectal administration, intracutaneous administration and intravenous administration are known, and among them oral administration is widely been adopted. However, in case of oral administration, there are drawbacks that a drug is susceptible to the first-pass effect in the liver after absorption of the drug and an unnecessarily high blood concentration is recognized for a while after the administration. Also, in oral administration many side effects such as gastrointestinal tract disorder, vomiting feeling and loss of appetite have been reported. Further, in recent aged society patients with reduced swallowing power increase, and preparations easy for administration clinically are desired.

Therefore, by dissolving these drawbacks of oral administration with the aim of percutaneous preparations for patients to take more easily with safety and persistence, development of such percutaneous administration preparations has actively been carried out, and the products are commercially available.

However, percutaneous absorbance of drugs in said percutaneous administration preparations is still insufficient in many cases, and the development of percutaneous administration preparations is difficult due to low percutaneous absorbance of most drugs, so it can hardly be said that the objects have sufficiently been attained. Namely, since normal skin has barrier function to prevent penetration of foreign substances, sufficient percutaneous absorption of a compounded pharmaceutically active ingredient is hardly attained in many cases.

Therefore, devices to increase percutaneous absorbance of a drug through a stratum corneum of skin has been needed, and compounding so-called percutaneous absorption promotors to a base has generally been tried. For example, as an absorption promotor combined with a lower alkyl amide, dimethylacetamide and ethyl, isopropyl, isopropyl palmitate or the like (US, 3,472,931, A), combining 2-pyrrolidone and an appropriate oil, and a straight chain fatty acid and alcohol ester ((US, 4,017,641, A), and combining lower alcohol and $C_7$-$C_{20}$ alcohol, $C_5$-$C_{30}$ fatty acid hydrocarbon, alcohol ester of $C_{19}$-$C_{26}$ fatty acid carboxylic acid, $C_{10}$-$C_{24}$ mono- or di-ether, $C_{11}$-$C_{15}$ ketone and water (JP, 61-249934, A) and the like have been proposed. However, it can hardly be said that these conventional absorption promotors and absorption promoting compositions are yet sufficiently safe for skin.

Further, as a percutaneous absorption preparation, methods of combining a drug and an organic acid have been reported. For example, a tape preparation combining betamethazone valerate and an organic acid to a natural rubber type adhesive (JP, 56-61312, A), a tape preparation combining a non-steroidal anti-inflammatory agent and an organic acid to an acrylic type adhesive agent (JP, 62-126119, A (US 4,740,374, A)), and a pap type preparation combining methyl salicylate as a pharmaceutically effective ingredient, an emulsifier, an organic acid, a plasticizer, a tackifying resin and water to a stylene-isoprene-stylene block copolymer (JP, 63-159315, A) and the like have already been proposed. However, the aim of using these organic acids in the above publications is to improve stability and solubility, and was a pH-adjusting agent, and because these drugs are acidic or neutral, they are not the preparations in which skin permeability of a physiologically active substance is improved via an ion-pair formation constructed by an organic acid of the present invention.

Further, a method to improve skin permeability of a basic physiologically active substance has been tried. For example, a tape preparation combining citric acid and isoproterenol hydrochloride to an acrylic type adhesive (JP, 63-79820, A), a tape preparation combining an organic acid and vinpocetine to an acrylic type adhesive (JP, 5-25039, A) and the like have been reported, though these have a problem of irritancy at the time of dissection, and that the released amount of the drugs do not give a sufficient effect for therapy.

The invention was made to dissolve the problems of the prior art described above, and makes it an object to provide a matrix type adhesive preparation. In WO, 96/16642, A, technology of an adhesive preparation in which an organic acid salt is contained in a salt type base drug is disclosed, though the effect of the particle size of the contained organic salt is not demonstrated.

### DISCLOSURE OF THE INVENTION

During extensive researches to solve these problems the inventors found out that comprising an organic acid salt of a particular particle size in adhesive preparations containing a base drug as a salt form improves solubility of the drug to skin via an ion-pair formation, and that it significantly improves skin permeability of the drug by enhancing partition coefficient to skin, and thus accomplished the invention. Specifically, in case of the mean diameter of a base drug and an organic acid salt contained was 100μm or less (this particle size indicates volume average particle size when measured by the use of a particle fineness analyzer) the effect was observed. Particularly, it was revealed that in a fat-soluble base, though the solubility of a drug and an organic acid salt was so bad they remain as powder in the preparation, percutaneous absorbance of the drug was greatly affected by the size of the particle diameter of the organic acid salt. In particular, as an organic acid salt, the effect of sodium acetate is high, and in this case the average particle size of 0. 1-10μm shows extremely excellent percutaneous drug-absorbance promoting effect.

Accordingly, the invention relates to an adhesive preparation comprising a base drug salt, and an organic acid salt in which the mean diameter is 0.1-100μm.

The invention also relates to the above adhesive preparation wherein the mean diameter of the organic acid salt is 0.1-10μm.

Further, the invention relates to the above adhesive preparation comprising the organic acid salt of 0.01-15% by weight.

The invention also relates to the above adhesive preparation comprising the base drug of 0. 1-20% by weight.

Furthermore, the invention relates to the above adhesive preparation characterized in that the organic acid is acetic acid.

And the invention relates to the above adhesive preparation characterized in that the organic acid is sodium acetate.

Further, adhesive preparations of the invention provide excellent skin permeability, skin irritancy and content stability of a drug and physical stability of a base.

The adhesive preparations of the invention are also preferably matrix type preparations.

**Brief Description of Drawing**

Fig. 1
Graph showing the results of skin permeability of Examples 1-3 and Comparative example 1.
Fig. 2
Graph showing the results of skin permeability of Examples 6-8 and Comparative examples 4-5.
Fig. 3
Graph showing the results of skin permeability of Examples 11-13 and Comparative examples 8-9.

In any drug, powders with a smaller particle size gave more excellent skin permeability to the drug.

**Embodiment of the Invention**

As the embodiment of the invention, the composition and the form in the adhesive layer of the adhesive preparations related to the invention is explained.

Illustrative of the organic acid salts used in the adhesive layer of the adhesive preparations according to the invention are respective water-soluble inorganic salts of aliphatic (mono, di, tri)carboxylic acids (e.g., acetic acid, propionic acid, isobutylic acid, caproic acid, lactic acid, maleic acid, pyruvic acid, oxalic acid, succinic acid, tartaric acid, etc.) , aromatic carboxylic acids (e.g., phthalic acid, salicylic acid, benzoic acid, acetyl salicylic acid, etc.), alkyl sulfonic acids (e.g., ethane sulfonic acid, propyl sulfonic acid, butane sulfonic acid, polyoxyetylene alkyl ether sulfonic acid, etc.), alkyl sulfonic acids (e.g., N-2-hydroxyethyl piperidine-N'-2-ethane sulfonic acid (hereafter abbreviated as HEPES) etc.) and cholic acid derivatives (e.g., dehydro cholic acid, etc.), and in particular sodium acetate is preferable. Further, these organic acid salts may be anhydrous or hydrated, though in case used in a hydrophobic adhesive layer, an anhydride is preferred.

Considering a sufficient permeable amount and irritancy to the skin for an adhesive preparation, these organic salts can be blended in an amount of preferably 0.01-15% by weight, more preferably 0.1-10% by weight, and most preferably 0.1-5% by weight based on the total weight of the composition in the adhesive preparation.

Also, in the case of a commercially available powder in which the average particle size of an organic acid salt is not less than about 100μm (in the case of sodium acetate, generally the average particle size of commercially available one is not less than about 500μm), it is ground in a preparation step to not more than 100μm to obtain an excellent percutaneous drug absorbance, and the average particle size is made preferably 0.1-100μm, more preferably 0.1-50μm, and most preferably 0.1-10μm. Namely, it is considered that an ion-pair formation is more sufficiently promoted as the particle size of powders becomes smaller. As a method to grind an organic acid salt, powders which are dry ground beforehand may be used, or not yet ground one is added to a solution containing other base ingredients and may be wet ground under stirring. For example, as a grinder mill of dry process, a supersonic jet grinder mill, Jet Mill, (manufactured by Nippon Pneumatic MFG Co., Ltd.) and the like, or as a grinder mill of wet process, an ultra-micro grinder mill, Micros, (manufactured by Nara Machinery Co., Ltd.) and the like can be used.

Further, as a drug used in the adhesive layer of the adhesive preparations of the invention, an inorganic salt forming an ion-pair with an organic acid or its salt, or any base drug salt formed by an organic acid is not limited particularly by their types; examples include hypnotic-sedative agents (fluazepam hydrochloride, rilmazafone hydrochloride, etc.), anti-inflammatory agents (butorphanol tartarate, persoxal citrate, etc.), excitation-analeptic agents (methanephetamine hydrochloride, methylphenidate hydrochloride, etc.), psychotropic agents (chlorpromazine hydrochloride, imipramine hydrochloride, etc.), local anesthetic agents (lidocain hydrochloride, procaine hydrochloride), agents for urinary organs (oxybutynin hydrochloride, etc.), skeletalmuscle relaxants (tizanidine hydrochloride, eperisone hydrochloride, pridinol mesilate, etc.), autonomic agents (carpronium chloride, neostigmine bromide, etc.), anti-Parkinson's disease agents (trihexyphenidyl hydrochloride, amantadine hydrochloride, etc.), antihistaminic agents (clemastine fumarate, diphenhydramine tannate, etc.), bronchodilator agents (tulobuterol hydrochloride, procaterol hydrochloride, etc.), cardiotonic agents (isoprenaline hydrochloride, dopamine hydrochloride, etc.), coronary dilators (diltiazem hydrochloride, verapamil hydrochloride, etc.), peripheral vasodilators (nicamate citrate, tolazoline hydrochloride, etc.), cardiovascular agents (flunarizine hydrochloride, nicardipine hydrochloride, etc.), antiarrhythmic agents (propranolol hydrochloride, alprenolol hydrochloride, etc.), antiallergic agents (ketotifen fumarate, azelastine hydrochloride, etc.), anti-dizziness agents (betahistine mesilate, difenidol hydrochloride, etc.), serotonin receptor antagonistic antiemetics, narcotic analgesic agents (morphine sulfate, fentanyl citrate, etc.).

Further, these drugs may be used alone or in combination of two or more of them, and any form of drug, an inorganic salt or an organic salt, are naturally included. Also, considering a sufficient permeable amount as adhesive preparations and irritancy to the skin such as rubor, drugs can be blended in an amount preferably of 0.01-15% by weight, and more preferably 0.1-20% by weight based on the total weight of the composition in the adhesive layer.

Any absorption promotor may be contained in the adhesive layer of the adhesive preparations of the invention, and as an absorption promotor, any compound in which absorption promoting effect is shown may be used. Examples include $C_6$-$C_{20}$ fatty acids, fatty alcohols, fatty acid esters or ethers, aromatic organic acids, aromatic alcohols, aromatic fatty acid esters or ethers (these may be saturated or unsaturated, and cyclic, straight or branched), furthermore lactic acid esters, acetic acid esters, monoterpene compounds, sesquiterpene compounds, Azone, Azone derivatives, glycerol fatty acid esters, sorbitan fatty acid esters (Span type), polysorbates (Tween type), polyethylene glycol fatty acid esters, polyoxyethylene hardened castor oils (HCO type), sucrose fatty acid esters and the like.

Specifically, caprylic acid, capric acid, lauric acid, myristic acid, palmitic acid, stearic acid, oleic acid, linoleic acid, linolenic acid, lauryl alcohol, myristyl alcohol, oleyl alcohol, cetyl alcohol, methyl laurate, isopropyl myristate, myristyl myristate, octyldecyl myristate, cetyl palmitate, salicylic acid, methyl salicylate, glycol salicylate, cinnamic acid, methyl cinnamate, cresol, cetyl lactate, ethyl acetate, propyl acetate, geraniol, thymol, eugenol, terpineol, 1-menthol, borneol, d-limonene, isoeugenol, isoborneol, nerol, dl-camphor, glycerol monolaurate, glycerol monooleate, sorbitan monolaurate, sucrose monolaurate, polysorbate 20, propylene glycol, polyethylene glycol monolaurate, polyethylene glycol monostearate, HCO-60, and 1-[2-(decylthio)ethyl]azacyclopentan -2-one (hereafter abbreviated as pyrothiodecane) are preferred, and lauryl alcohol, 1-menthol, propylene glycol and pyrothiodecane are particularly preferred.

Considering a sufficient permeable amount as adhesive preparations and irritancy to the skin such as rubor and edema, such absorption promotors can be blended in an amount preferably of 0.01-20% by weight, more preferably 0.05-10% by weight and most preferably 0.1-5% by weight based on the total weight of the composition in the adhesive preparations.

As a fat soluble hydrophobic polymer used in the adhesive layer of the adhesive preparations of the invention, examples include styrene-isoprene-styrene block copolymer (hereinafter abbreviated as SIS), isoprene rubber, polyisobutylene (hereinafter abbreviated as PIB), styrene-butadiene-styrene block copolymer (hereinafter abbreviated as SBS), styrene-butadiene rubber (hereinafter abbreviated as SBR), acrylic type polymer (copolymer of at least two types from 2-ethylhexyl acrylate, vinyl acetate, methacrylate, methoxyethyl acrylate and acrylic acid). In particular, SIS, PIB or blends thereof, and acrylic type polymer are preferred.

Considering formation of the adhesive layer and sufficient permeability, the blended amount of such hydrophobic polymers based on the total weight of the composition in the adhesive layer can be 10-60% by weight, preferably 15-50% by weight, more preferably 18-40% by weight in SIS, PIB or the like. Similarly, it can be 10-98% by weight, preferably 20-98% by weight, more preferably 30-98% by weight in acrylic type polymer.

As a tackifying resin used in the adhesive layer of the adhesive preparations of the invention, examples include rosin derivatives (e.g,. rosin, glycerol esters of rosin, hydorogenated rosin, glycerol esters of hydorogenated rosin, pentaerythritol esters of rosin, etc.), alicyclic saturated hydrocarbon resins, aliphatic hydrocarbon resins, terpene resins, maleic acid resins and the like. In particular, glycerol esters of hydorogenated rosin, alicyclic saturated hydrocarbon resins, aliphatic hydrocarbon resins and terpene resins are preferred.

Considering sufficient adhesive strength as the adhesive preparations and irritancy to the skin at the time of dissection, the compounded amount of such tackifying resins based on the total weight of the composition in the adhesive layer can be 10-70% by weight, preferably 15-60% by weight, and more preferably 20-50%.

As a plasticizer used in the adhesive layer of the adhesive preparations of the invention, examples include petroleum oils (e.g., paraffin type process oil, naphthalene type process oil, aromatic type process oil, etc.), squalene, vegetable oils (olive oil, camellia oil, castor oil, tall oil, peanut oil), dibasic acid esters (e.g., dibutyl phthalate, dioctyl phthalate, etc.), liquid rubber (e.g., polybutene, liquid isoprene rubber), diethyleneglycol, polyethylene glycol, glycol salicylate, propylene glycol, dipropylene glycol, crotamiton and the like. In particular, liquid paraffin, liquid polybutene, glycol salicylate and crotamiton are preferred.

Considering sufficient permeability and the maintenance of sufficient agglutinative strength as adhesive preparations, the blended amount of such a tackifying resin based on the total weight of the composition in the adhesive layer can be 10-70% by weight, preferably 15-60% by weight, and more preferably 20-50%.

Also, as required, antioxidants, fillers, cross-linking agents, preservatives or UV absorbers can be used. As antioxidants, tocopherol and its ester derivatives, ascorbic acid, ascorbic acid-stearic acid ester, nordihydroguaretic acid, dibutyl hydroxy toluene (BHT), butyl hydroxy anisole and the like are desirable. As fillers, calcium carbonate, magnesium carbonate, silicate (e.g., aluminum silicate, magnesium silicate, etc.), silicic acid, barium sulfate, calcium sulfate, calcium zincate, zinc oxide, titanic oxide and the like are desirable. As cross-linking agents, thermosetting resins such as amino resins, phenol resins, epoxy resins, alkyd resins, unsaturated polyesters, etc., isocyanate compounds, block isocyanate compounds, organic type cross-linking agents, and inorganic type cross-linking agents such as metals or metal compounds, are desirable. As preservatives, ethyl p-oxybenzoate, propyl p-oxybenzoate, butyl p-oxybenzoate and the like are desirable. As UV absorbers, p-amino benzoic acid derivatives, anthranilic acid derivatives, salicylic acid derivatives, coumarin derivatives, amino acid type compounds, imidazoline derivatives, pyrimidine derivatives, dioxane derivatives and the like are desirable.

Such antioxidants, fillers, cross-linking agents, preservatives or UV absorbers can be blended in total preferably in an amount of not more than 10% by weight, more preferably not more than 5% by weight and most preferably not more than 2% by weight based on the total weight of the composition in the adhesive layer of the adhesive preparations.

The adhesive layer having such a composition can be prepared by any method. For example, a base composition containing a drug is heat-melted, coated on removable paper or a backing, followed by affixing each to the backing or the removable paper to give the present preparations. Also, base ingredients containing a drug are dissolved in solvent such as toluene, hexane or ethyl acetate, spreaded on removable paper or a backing, dried to remove solvent, followed by affixing to the backing or the removable paper to give the present preparations.

Further, the adhesive preparations of the invention may take any other structures and materials for each constituent, if the adhesive layer has the above composition containing the organic acid salt and the drug.

For example, in addition to the above adhesive layer the adhesive preparations of the invention can comprise of a backing layer to support it and a removable paper layer set on the adhesive layer.

As to the backing layer, an elastic or a non-elastic backing can be used. For example, it can be selected from fabric, polyurethane, polyester, polyvinyl acetate, polyvinylidene chloride, polyethylene, polyethylene terephthalate, aluminum sheet and the like, or composite materials thereof.

**Example**

In the following, the invention is explained in more detail by the examples. The invention, however, is not limited to these examples, and various changes may be made without departing from the spirit of the invention. Further, in the examples, all "%"s mean % by weight.

Example 1

| | |
|---|---|
| Styrene-isoprene-styrene block copolymer (SIS) | 24.0% |
| Alicyclic saturated hydrocarbon (Arkon P-100) | 29.5% |
| Liquid paraffin (Crystol 352) | 41.0% |
| Pyrothiodecane | 2.0% |

(continued)

| | |
|---|---|
| Sodium acetate | 1.5% |
| Ketotifen fumarate | 1.5% |
| Butyl hydroxy toluene [BHT (Yoshinox)] | 0.5% |
| Total amount | 100.0% |

Sodium acetate (average particle size 7 µm) ground by Jet Mill beforehand was used, and the polymer contained was heat-melted. The components were coated on removable paper, followed by affixing said removable paper to the backing to give the matrix adhesive preparation of the invention.

Example 2

Sodium acetate (average particle size 43 µm) ground using a mortar beforehand was used, and the other ingredients and the preparation steps were the same as those of Example 1.

Example 3

Sodium acetate (average particle size 91 µm) ground using a mortar beforehand was used, and the other ingredients and the preparation steps were the same as those of Example 1.

Example 4

| | |
|---|---|
| SIS | 22.5% |
| Alicyclic saturated hydrocarbon (Arkon P-85) | 27.5% |
| Liquid paraffin | 32.0% |
| Lauryl alcohol | 5.0% |
| Sodium acetate | 5.0% |
| Lidocain hydrochloride | 7.5% |
| BHT | 0.5% |
| Total amount | 100.0% |

Sodium acetate (average particle size 43 µm) ground using mortar beforehand was used, and the polymer contained was heat-melted. The components were coated on removable paper, followed by affixing the removal paper to the backing to give the matrix adhesive preparation of the invention.

Example 5

| | |
|---|---|
| SIS | 15.5% |
| Polyisobutylene (PIB) | 6.5% |
| Alicyclic saturated hydrocarbon (Arcon P-100) | 33.0% |
| Liquid paraffin | 31.5% |

(continued)

| | |
|---|---|
| Crotamiton | 5.0% |
| Sodium acetate | 3.0% |
| Oxybutynin hydrochloride | 5.0% |
| BHT | 0.5% |
| Total amount | 100.0% |

Sodium acetate (average particle size 43 μm) ground using a mortar beforehand was used, and the polymer contained was heat-melted. The components were coated on removable paper, followed by affixing said removable paper to the backing to give the matrix adhesive preparation of the invention.

Example 6

| | |
|---|---|
| SIS | 26.0% |
| Hydrogenated rosin ester | 35.0% |
| Liquid paraffin | 28.6% |
| Crotamiton | 5.0% |
| Pyrothiodecane | 3.0% |
| Sodium acetate | 0.4% |
| Tizanidine hydrochloride | 1.5% |
| BHT | 0.5% |
| Total amount | 100.0% |

All the compositions including sodium acetate (average particle size 7 μm) ground by Jet Mill beforehand were dissolved in toluene, coated on removable paper, dried to remove solvent, followed by affixing said removable paper to the backing to give the matrix adhesive preparation of the invention.

Example 7

Sodium acetate (average particle size 43 μm) ground using a mortar beforehand was used, and the other ingredients and the preparation steps were the same as those of Example 6.

Example 8

Sodium acetate (average particle size 91 μm) ground using a mortar beforehand was used, and the other ingredients and the preparation steps were the same as those of Example 6.

Example 9

| | |
|---|---|
| PIB | 28.5% |
| Rosin ester | 29.5% |

(continued)

| Liquid paraffin | 33.5% |
|---|---|
| 1-Menthol | 3.0% |
| Sodium acetate | 2.0% |
| Pridinol mesylate | 3.0% |
| BHT | 0.5% |
| Total amount | 100.0% |

All the compositions including sodium acetate (average particle size 7 μm) ground by Jet Mill beforehand was dissolved in toluene, coated on removable paper, dried to remove solvent, followed by affixing said removable paper to the backing to give the matrix adhesive preparation of the invention.

Example 10

| SIS | 20.5% |
|---|---|
| PIB | 5.5% |
| Terpene resin (YS resin Px1000) | 21.0% |
| Liquid paraffin | 44.0% |
| Propylene glycol | 2.5% |
| Sodium acetate | 3.0% |
| Tulobuterol hydrochloride | 3.0% |
| BHT | 0.5% |
| Total amount | 100.0% |

All the compositions including sodium acetate (average particle size 91μm) ground using a mortar beforehand were dissolved in toluene, coated on removable paper, dried to remove solvent, followed by affixing said removable paper to the backing to give the matrix adhesive preparation of the invention.

Example 11

| SIS | 24.0% |
|---|---|
| Alicyclic saturated hydrocarbon resin (Arcon P-100) | 30.0% |
| Liquid paraffin | 38.0% |
| pyrothiodecane | 3.0% |
| Sodium acetate | 1.5% |
| Fentanyl citrate | 3.0% |
| BHT | 0.5% |
| Total amount | 100.0% |

Among the above ingredients, all the powder ingredients (sodium acetate, fentanyl citrate) were contained in liquid paraffin, ground by Micros to make the mean diameter 10 μm or smaller. This and the other ingredients were dissolved in toluene, coated on removable paper, dried to remove solvent, followed by affixing said removable paper to the backing to give the matrix adhesive preparation of the invention.

Example 12

Sodium acetate (average particle size 43 μm) ground using a mortar beforehand was used, and the other ingredients were the same as those of Example 11, whereby the preparation was formulated using a stirrer which has no grinder function.

Example 13

Sodium acetate (average particle size 91 μm) ground using a mortar beforehand was used, and the other ingredients were same as those of Example 11, whereby the preparation was formulated using a stirrer which has no grinder function.

Example 14

| SIS | 21.0% |
| --- | --- |
| PIB | 9.5% |
| Aliphatic type hydrocarbon resin (Quintone B170) | 25.0% |
| Polybutene | 5.5% |
| Liquid paraffin | 28.5% |
| Propylene glycol | 3.0% |
| Sodium acetate | 2.0% |
| Propranolol hydrochloride | 5.0% |
| BHT | 0.5% |
| Total amount | 100.0% |

Among the above ingredients, all the powder ingredients (sodium acetate, propranolol hydrochloride) were contained in liquid paraffin, ground by Micros to make the mean diameter 50 μm or smaller. This and the other ingredients were dissolved in toluene, coated on removable paper, dried to remove solvent, followed by affixing said removable paper to the backing to give the matrix adhesive preparation of the invention.

Example 15

| SIS | 18.0% |
| --- | --- |
| PIB | 6.0% |
| Alicyclic saturated hydrocarbon resin (Arkon P-100) | 31.5% |
| Liquid paraffin | 30.5% |
| Lauryl alcohol | 5.0% |

(continued)

| | |
|---|---|
| Sodium acetate | 5.0% |
| Azelastine hydrochloride | 3.5% |
| BHT | 0.5% |
| Total amount | 100.0% |

Among the above ingredients, all the powder ingredients (sodium acetate, azelastine hydrochloride) were contained in liquid paraffin, ground by Micros to make the mean diameter 100 μm or smaller. This and the other ingredients were dissolved in toluene, coated on removable paper, dried to remove solvent, followed by affixing said removable paper to the backing to give the matrix adhesive preparation of the invention.

Example 16

| | |
|---|---|
| Acrylic polymer (Nissetsu PE-300: Nippon Carbide Industries Co., INC.) | 92.0% |
| Cross-linking agent (Nissetsu CK-100: Nippon Carbide Industries Co., INC.) | 0.5% |
| 1-Menthol | 3.0% |
| Sodium acetate | 1.5% |
| Fentanyl citrate | 3.0% |
| Total amount | 100.0% |

Among the above ingredients, 1-menthol, fentanyl citrate and sodium acetate (average particle size 7 μm) ground by Jet Mill beforehand were added to ethanol, dissolved under stirring at room temperature. The mixture was then added with an ethyl acetate solution of the acrylic polymer and with the cross-linking agent, stirred, coated on removable paper, dried to remove solvent, followed by a heat cross-linking and by affixing said removable paper to the backing to give the matrix adhesive preparation of the invention.

**COMPARATIVE EXAMPLE**

Comparative example 1

Sodium acetate (average particle size 535 μm) not yet ground was used, and the other ingredients and the preparation steps were the same as those of Example 1.

Comparative example 2

Sodium acetate (average particle size 535 μm) not yet ground was used, and the other ingredients and the preparation steps were the same as those of Example 4.

Comparative example 3

Sodium acetate (average particle size 535 μm) not yet ground was used, and the other ingredients and the preparation steps were the same as those of Example 5.

Comparative example 4

Sodium acetate (average particle size 200 μm) ground using a mortar beforehand was used, and the other ingredients and the preparation steps were the same as those of Example 6.

Comparative example 5

Sodium acetate (average particle size 535 μm) not yet ground was used, and the other ingredients and the preparation steps were the same as those of Example 6.

Comparative example 6

Sodium acetate (average particle size 535 μm) not yet ground was used, and the other ingredients and the preparation steps were the same as those of Example 9.

Comparative example 7

Sodium acetate (average particle size 535 μm) not yet ground was used, and the other ingredients and the preparation steps were the same as those of Example 10.

Comparative example 8

Sodium acetate (average particle size 139 μm) ground using a mortar beforehand was used, and the other ingredients were same as those of Example 11, whereby the preparation was formulated using a stirrer which has no grinder function.

Comparative example 9

Sodium acetate (average particle size 535 μm) not yet ground was used, and the other ingredients were the same as those of Example 11, whereby the preparation was formulated using a stirrer which has no grinder function.

Comparative example 10

Sodium acetate (average particle size 535 μm) not yet ground was used, and the other ingredients were the same as those of Example 14, whereby the preparation was formulated out using a stirrer which has no grinder function.

Comparative example 11

Sodium acetate (average particle size 535 μm) not yet ground was used, and the other ingredients were the same as those of Example 15, whereby the preparation was formulated using a stirrer which has no grinder function.

Comparative example 12

Sodium acetate (average particle size 535 μm) not yet ground was used, and the other ingredients and the preparation steps were the same as those of Example 16.

(Skin permeability test on hairless mice)

Dorsal skin of a hairless mouse was stripped, and the dermal side was placed to the receptor layer side and installed in a flow-through cell (5 cm$^2$) around whose periphery warm water at 37° C was circulated. Each of the adhesive preparations obtained in the examples 1-3, 6-8 and 11-13 as well as the comparative examples 1, 4, 5, 8 and 9 was coated on the stratum corneum side, and sampling was carried out every one hour (or 2 hours) for 12 hours (or 18 hours, 24 hours) at the rate of 5 ml/hour using the physiological saline in the receptor layer. As to the receiver solutions obtained at every hour, the flow amounts were accurately measured, and the drug concentrations were measured by high-performance liquid chromatography, followed by calculation of the permeation rate per hour to determine the skin permeability rate according to the following equation.

$$\text{Skin permeability rate } (\mu g/cm^2/hr) = \{\text{sample concentration } (\mu g/ml) \times \text{flow amount (ml)}\} / \text{applied area of the preparation } (cm^2)$$

**Industrial Applicability**

According to the adhesive preparations of the invention, drugs can be efficiently absorbed into circulating blood via skin. Also, side effects of the gastrointestinal system observed in case of oral administration, and side effects in the central nervous system which can occur due to a rapid increase of the blood concentration can be avoided. Further, they are extremely low in irritancy to the skin. Therefore, these are very effective as external preparations aiming at percutaneous application.

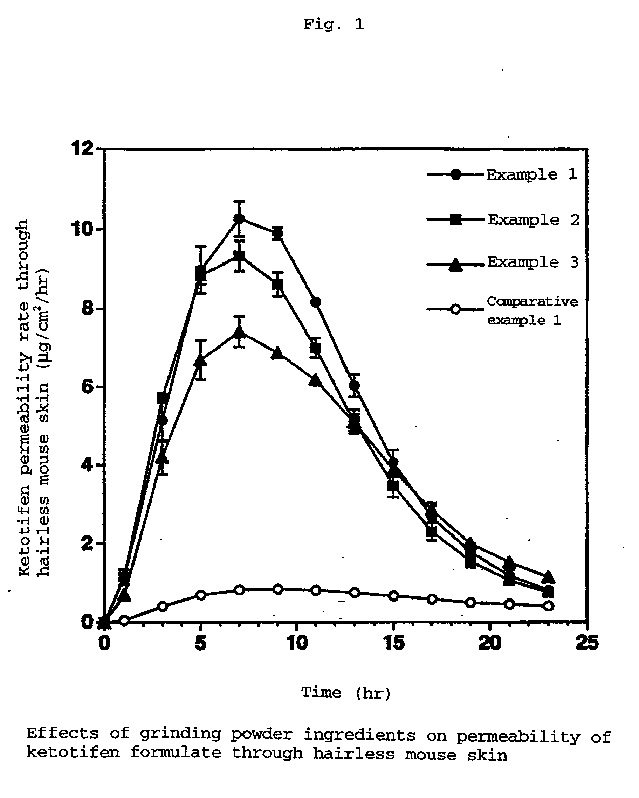

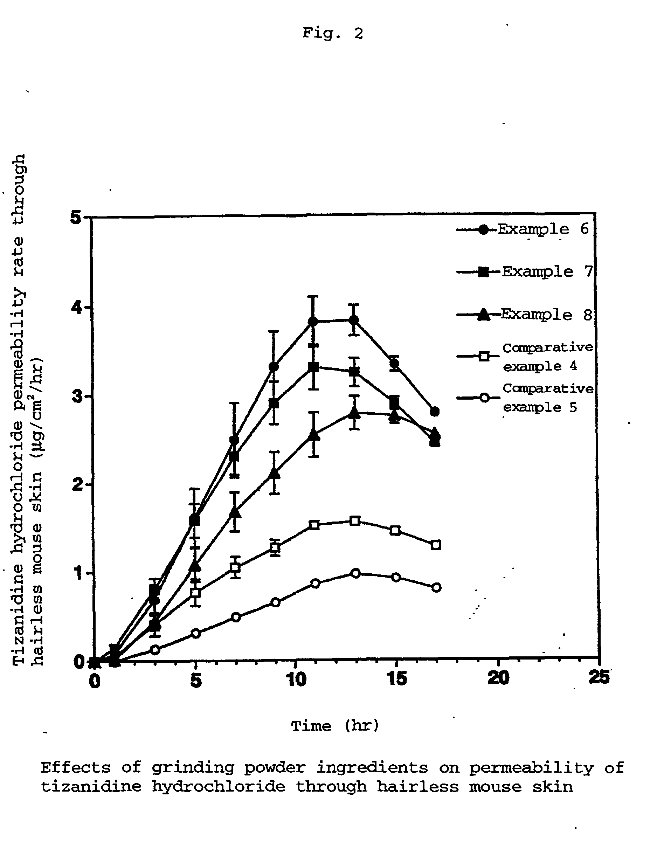

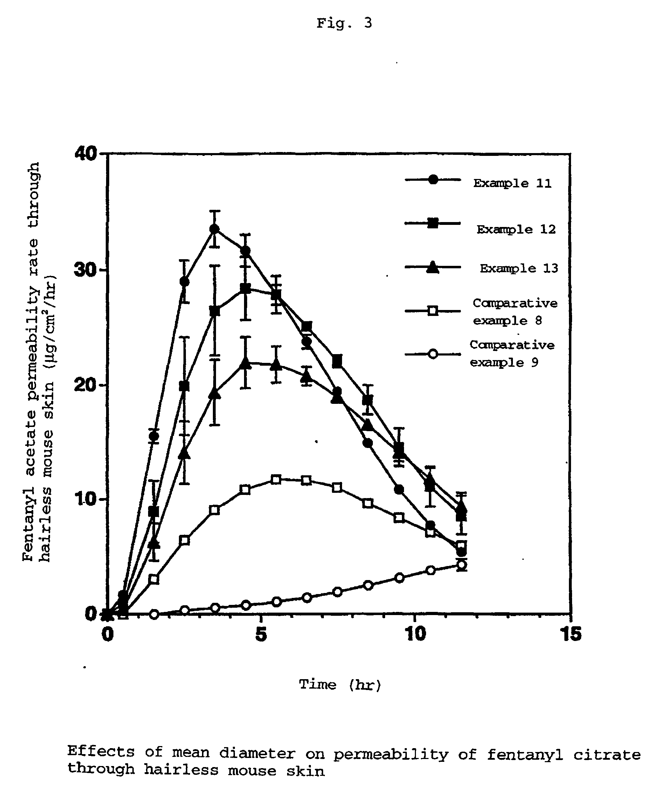

**Claims**

1. An adhesive preparation comprising a base drug salt and an organic acid salt in the form of powder having a mean diameter of 0.1-100 μm.

2. The adhesive preparation according to claim 1, wherein the mean diameter of the organic acid salt is 0.1-10 μm.

3. The adhesive preparation according to claim 1 or 2 comprising the organic acid salt of 0.01-15% by weight.

4. The adhesive preparation according to claim 1 or 2 comprising the base drug salt of 0.1-20% by weight.

5. The adhesive preparation according to any one of claims 1 to 3, characterized in that the organic acid salt is an acetic acid salt.

6. The adhesive preparation according to claim 5, characterized in that the organic acid salt is sodium acetate.

Fig. 1

Effects of grinding powder ingredients on permeability of
ketotifen formulate through hairless mouse skin

Fig. 2

Effects of grinding powder ingredients on permeability of tizanidine hydrochloride through hairless mouse skin

Fig. 3

Effects of mean diameter on permeability of fentanyl citrate through hairless mouse skin

# INTERNATIONAL SEARCH REPORT

| International application No. |
|---|
| PCT/JP99/01868 |

| A. CLASSIFICATION OF SUBJECT MATTER |
|---|
| Int.Cl⁶ A61K9/70, A61K45/08, A61K31/135, 31/14, 31/165, 31/215, 31/415, 31/445, 31/55 |
| According to International Patent Classification (IPC) or to both national classification and IPC |

| B. FIELDS SEARCHED |
|---|
| Minimum documentation searched (classification system followed by classification symbols) |
| Int.Cl⁶ A61K9/70, A61K45/08, A61K31/135, 31/14, 31/165, 31/215, 31/415, 31/445, 31/55 |

| Documentation searched other than minimum documentation to the extent that such documents are included in the fields searched |
|---|
| |

| Electronic data base consulted during the international search (name of data base and, where practicable, search terms used) |
|---|
| |

| C. DOCUMENTS CONSIDERED TO BE RELEVANT | | |
|---|---|---|
| Category* | Citation of document, with indication, where appropriate, of the relevant passages | Relevant to claim No. |
| X<br>Y | JP, 10-45570, A (Hisamitsu Pharmaceutical Co., Inc.),<br>17 February, 1998 (17. 02. 98),<br>Full text & WO, 97/42952, A1 & EP, 842662, A1 | 1, 3-6<br>2 |
| X | JP, 10-1432, A (Sekisui Chemical Co., Ltd.),<br>6 January, 1998 (06. 01. 98),<br>Full text (Family: none) | 1-4 |
| X<br>Y | JP, 8-157365, A (Hisamitsu Pharmaceutical Co., Inc.),<br>18 June, 1996 (18. 06. 96),<br>Full text & WO, 96/16642, A1<br>& EP, 788792, A1 & US, 5866157, A | 1, 3-6<br>2 |
| X<br>Y | JP, 3-261722, A (Sekisui Chemical Co., Ltd.),<br>21 November, 1991 (21. 11. 91),<br>Full text (Family: none) | 1, 3, 4<br>2 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 23 June, 1999 (23. 06. 99) | 6 July, 1999 (06. 07. 99) |

| Name and mailing address of the ISA/<br>Japanese Patent Office | Authorized officer |
|---|---|
| Facsimile No. | Telephone No. |

Form PCT/ISA/210 (second sheet) (July 1992)

EP 1 074 251 A1

## INTERNATIONAL SEARCH REPORT

International application No.

PCT/JP99/01868

C (Continuation). DOCUMENTS CONSIDERED TO BE RELEVANT

| Category* | Citation of document, with indication, where appropriate, of the relevant passages | Relevant to claim No. |
|---|---|---|
| Y | JP, 63-79820, A (Nitto Electric Industrial Co., Ltd.), 9 April, 1988 (09. 04. 88), Page 3, upper left column, upper right column (Family: none) | 2 |

Form PCT/ISA/210 (continuation of second sheet) (July 1992)